# EUROPÄISCHE PATENTSCHRIFT

(11) **EP 0 622 397 B1**
(45) Veröffentlichungstag und Bekanntmachung des Hinweises auf die Patenterteilung: **19.06.1996**
(21) Anmeldenummer: 94103203.9
(22) Anmeldetag: 03.03.1994
(51) Int. Cl.: C08J 3/03, C08L 83/04

(54) **Emulsionen polare Gruppen enthaltender Organopolysiloxane mit Alkylpolyglykosiden als Emulgatoren**
Emulsions of polar groups-containing organopolysiloxanes with alkylpolyglycosides as emulsifiers
Emulsions d'organopolysiloxanes à groupes polaires contenant des alkylpolyglycosides comme émulsifiants

(30) Priorität: 04.03.1993 DE 4306796
(43) Veröffentlichungstag der Anmeldung: 02.11.1994
(73) Patentinhaber: Wacker-Chemie GmbH, D-81737 München (DE)
(72) Erfinder: Geck, Michael, Dr., D-84489 Burghausen (DE); Deubzer, Bernward, Dr., D-84489 Burghausen (DE); Baumgartner, Christine, D-84508 Burgkirchen (DE); Lautenschlager, Hans-Jürgen, Dr., D-84533 Haiming (DE); Sejpka, Johann, Dr., D-84533 Marktl (DE)

(56) Entgegenhaltungen:
- EP-A- 0 337 354
- EP-A- 0 398 177
- WO-A-92/08439
- DE-A- 4 131 551

## Beschreibung

Die vorliegende Erfindung betrifft wäßrige Emulsionen polare Gruppen enthaltender Organopolysiloxane mit Alkylpolyglykosiden als Emulgatoren.

Für viele Anwendungen, wie zum Beispiel bei der Behandlung von Fasermaterialien aller Art werden wäßrige Emulsionen polare Gruppen enthaltender Organopolysiloxane mit hohen Fremdelektrolytkonzentrationen versetzt. Beispielsweise werden bei der Hochveredelung von Baumwoll- und Baumwollmischgeweben Emulsionen von amino- und ammoniumfunktionellen Organopolysiloxanen mit großen Mengen an anorganischen Salzen, wie Magnesium- und Natriumsalzen, belastet. Dadurch darf die Emulsion aber nicht brechen.

Beispielsweise sind in US-A-5,057,572 und EP-A-442 098 feinteilige, gegenüber Fremdionen für viele Zwecke hinreichend stabile wäßrige Emulsionen polare Gruppen enthaltender aminoalkylsubstituierter Organopolysiloxane mit Alkylpolyglykolethern, insbesondere mit Ethoxylaten verzweigter Alkohole, als Emulgatoren beschrieben. Die aquatische Toxizität von Alkoholethoxylaten ist jedoch beträchtlich. Bei Fettalkoholethoxylaten mit linearen Alkylketten erfolgen der biologische Primär- und Totalabbau zwar relativ rasch, bei verzweigtkettigen Alkoholethoxylaten dagegen unzureichend langsam.

Bei den meisten Anwendungen von wäßrigen Emulsionen von Organopolysiloxanen wird jedoch nur der Organopolysiloxanbestandteil genutzt und die wäßrige Phase, die die Hauptmenge an Tensiden enthält, dem Abwasser zugeführt.

In der EP-A-418 479 sind stabile wäßrige Emulsionen von Polydimethylsiloxanölen und Polydiphenylsiloxanölen mit Alkylpolyglykosiden als Emulgatoren beschrieben. Die Alkylpolyglykoside sind zwar sehr gut biologisch abbaubar und weisen eine geringe Toxizität auf, jedoch sind diese Emulsionen gegenüber Fremdelektrolyten ebenso wie Emulsionen mit Alkoholethoxylaten als Emulgatoren nur mäßig stabil und aufgrund fehlender polarer Gruppen an den Siloxanen nur für bestimmte Zwecke einsetzbar.

In der WO-92/08439 sind Waschzubereitungen für die Haut- und Haarpflege beschrieben, die als Hauptkomponente und Tensid Alkylpolyglykoside und als Pflegebestandteil polare Gruppen aufweisende Organopolysiloxane enthalten. Das Gewichtsverhältnis der Organopolysiloxane zu den Alkylpolyglykosiden soll hier nicht größer als 1 sein.

Es war Aufgabe der vorliegenden Erfindung, in Anwesenheit von Fremdelektrolyten stabile wäßrige Emulsionen polare Gruppen enthaltender Organopolysiloxane mit möglichst geringen Mengen an Emulgatoren bereitzustellen, wobei die Emulgatoren sehr gut biologisch abbaubar sind und eine geringe Toxizität aufweisen.

Die vorstehend beschriebenen Aufgaben werden durch die vorliegende Erfindung gelöst durch wäßrige Emulsionen auf der Grundlage von
a) 100 Gewichtsteilen an Organopolysiloxanen, welche polare Gruppen an Si-C-gebundenen Kohlenwasserstoffresten aufweisen, und
b) höchstens 50 Gewichtsteilen an Alkylpolyglykosiden.

Methyl- oder Phenylgruppen sind nicht unter die polare Gruppen aufweisenden Si-C-gebundenen Kohlenwasserstoffreste zu rechnen.

Im Gegensatz zu den wäßrigen Emulsionen von Polydimethylsiloxanölen und Polydiphenylsiloxanölen mit Alkylpolyglykosiden weisen die erfindungsgemäßen Emulsionen eine höhere Stabilität gegenüber Fremdelektrolyten, wie Magnesium- und Natriumsalzen, auf als entsprechende Emulsionen, bei denen Alkylpolyglykolether als Emulgatoren verwendet werden.

Die erfindungsgemäßen Emulsionen enthalten relativ geringe Mengen an Emulgatoren, insbesondere höchstens 40 Gewichtsteile an Alkylpolyglykosiden (b) pro 100 Gewichtsteile polare Gruppen enthaltender Organopolysiloxane (a), so daß die Abwasserbelastung auch dadurch reduziert wird. Vorzugsweise werden jedoch pro 100 Gewichtsteile Organopolysiloxane (a) mindestens 5, insbesondere 10, Gewichtsteile an Alkylpolyglykosiden (b) eingesetzt, um eine gute Stabilität der Emulsionen zu gewährleisten.

Die erfindungsgemäßen Emulsionen weisen eine diskontinuierliche Ölphase, welche die polare Gruppen enthaltenden Organopolysiloxane (a) enthält, und eine kontinuierliche Wasserphase auf.

Die Anteile des Organopolysiloxans (a) und der kontinuierlichen Wasserphase können in weiten Bereichen variiert werden, je nachdem, welcher Festgehalt in den erfindungsgemäßen Emulsionen und Mikroemulsionen angestrebt wird. Vorzugsweise liegt der Anteil des Organopolysiloxans (a) zwischen 5 und 60 Gewichtsprozent, insbesondere jedoch zwischen 10 und 40 Gewichtsprozent, des Gesamtgewichts der Emulsion.

Beispiele für in den Organopolysiloxanen (a) enthaltenen polare Gruppen, die die Si-C-gebundenen Kohlenwasserstoffreste aufweisen, sind Amino-, Ammonium-, Epoxy-, Hydroxy-, Amido-, Mercapto-, Carboxy- und/oder Sulfonsäuregruppen, deren Salze oder Ester.

Vorzugsweise weisen die Organopolysiloxane (a) die allgemeine Formel (I)

RₙR'ₘSiO_{(4-n-m)/2} (I)

auf, worin
- **R**: gleiche oder verschiedene, gegebenenfalls substituierte Kohlenwasserstoffreste oder Kohlenwasserstoffoxyreste mit jeweils 1 bis 18 Kohlenstoffatomen, Wasserstoffatome oder Hydroxyreste,
- **R'**: gleiche oder verschiedene, Si-C-gebundene, polare Gruppen enthaltende substituierte Kohlenwasserstoffreste,

- **n**: eine ganze Zahl im Wert von 0, 1, 2 oder 3,
- **m**: eine ganze Zahl im Wert von 0, 1, 2 oder 3
bedeuten, und die Summe n+m einen durchschnittlichen Wert von 1,8 bis 2,2 besitzt und m so gewählt ist, daß das Polyorganosiloxan mindestens einen Rest **R'** aufweist.

Vorzugsweise besitzt die Summe **n** + **m** einen durchschnittlichen Wert von 1,9 bis 2,1.

Beispiele für Kohlenwasserstoffreste **R** sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Alkenylreste, wie der Vinyl-, Allyl- und der 5-Hexen-1-ylrest; Cycloalkylreste, wie Cyclopentyl-, Cyclohexyl-, Cycloheptylreste und Methylcyclohexylreste; Arylreste, wie der Phenyl-, Naphthyl- und Anthryl- und Phenanthrylrest; Alkarylreste, wie o-, m-, p-Tolylreste, Xylylreste und Ethylphenylreste; Aralkylreste, wie der Benzylrest, der alpha- und der β-Phenylethylrest.

Beispiele für gegebenfalls substituierte Kohlenwasserstoffoxyreste **R** sind über ein direkt an ein Siliciumatom gebundenes Sauerstoffatom gebundene substituierte und unsubstituierte Kohlenwasserstoffreste **R** gemäß den vorstehend genannten Beispielen, insbesondere Alkoxyreste mit 1 bis 18 Kohlenstoffatomen und Phenoxyreste, speziell der Methoxy-, Ethoxy-, n-Propoxy-, iso-Propoxy- und Phenoxyrest. Vorzugsweise sind höchstens 5 % der Reste **R** gegebenfalls substituierte Kohlenwasserstoffoxyreste.

Beispiele für bevorzugte aminofunktionelle Reste **R'** sind Reste der allgemeinen Formel (II)

-R¹-[NR²(CH₂)ₐ]_{b}NHR², (II)

und deren durch Umsetzung mit Mineral- oder Carbonsäuren herstellbare Ammoniumsalze, wobei
- **R**^{**1**}: einen zweiwertigen C₁- bis C₁₈-Kohlenwasserstoffrest bedeutet,
- **R**^{**2**}: ein Wasserstoffatom oder einen gegebenenfalls Fluor-, Chlor- oder Brom-substituierten C₁- bis C₁₈-Kohlenwasserstoffrest bedeutet,

- **a**: die Werte 2, 3, 4, 5 oder 6 hat und
- **b**: die Werte 0, 1, 2, 3 oder 4 hat.

Beispiele für die zweiwertigen C₁- bis C₁₈-Kohlenwasserstoffreste R¹ sind gesättigte gerad- oder verzweigtkettige oder cyclische Alkylenreste wie der Methylen- und Ethylenrest sowie Propylen-, Butylen-, Pentylen-, Hexylen-, 2-Methylpropylen-, Cyclohexylen- und Octadecylenreste oder ungesättigte Alkylen- oder Arylenreste wie der Hexenylenrest und Phenylenreste, wobei der n-Propylenrest und der 2-Methylpropylenrest besonders bevorzugt sind.

Beispiele für die Kohlenwasserstoffreste R² sind die für R angegebenen Beispiele. Beispiele für halogensubstituierte Kohlenwasserstoffreste R² sind Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m- und p-Chlorphenylrest.

In der vorstehenden allgemeinen Formel (II) bedeuten vorzugsweise
- **R**^{**1**}: einen zweiwertigen C₂- bis C₆-Kohlenwasserstoffrest,
- **R**^{**2**}: ein Wasserstoffatom, einen Methyl- oder Cyclohexylrest,
- **a**: die Werte 2 oder 3 und
- **b**: die Werte 0 oder 1.

Besonders bevorzugt sind lineare Polydimethylsiloxane, die gegebenenfalls als Reste R neben Methylresten höchstens 5 % C₁- bis C₃-Alkoxy- oder Hydroxyendgruppen aufweisen. Vorzugsweise weisen diese Polydimethylsiloxane als Reste R' die Reste H₂N(CH₂)₂NH (CH₂)₃-, H₂N(CH₂)₂NHCH₂CH(CH₃)CH₂-, H₂N(CH₂)₃-, 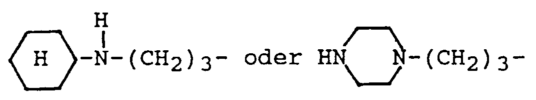 auf.

Beispiele für Mineralsäuren, die sich mit den vorstehend genannten aminofunktionellen Kohlenwasserstoffresten zu den entsprechenden ammoniumfunktionellen Resten umsetzen lassen, sind Salz-, Perchlor-, Schwefel-, schweflige, Salpeter-, salpetrige, Fluß-, Phosphor-, Diphosphor- und Polyphosphorsäuren. Beispiele für geeignete Carbonsäuren sind Ameisen-, Essig-, Propion-, Butansäuren, Citronensäure, Trichlor-, Dichlor- und Chloressigsäure, Trifluoressigsäure, Cyanessigsäure, Phenylessigsäure, Benzoesäure, m- und p-Nitrobenzoesäure, Oxalsäure, Malonsäure und Milchsäure. Besonders bevorzugt sind die mit Essigsäure erhältlichen

ammoniumfunktionellen Kohlenwasserstoffreste. Beispiele für amidofunktionelle Reste sind der γ-Acetamidopropylrest, teil- oder vollacetylierte β-Aminoethyl-γ-aminopropylreste.

Beispiele für epoxyfunktionelle Reste **R'** sind Reste der allgemeinen Formeln (III) und (IV) 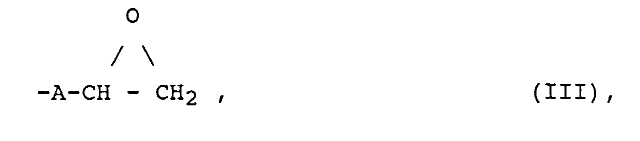 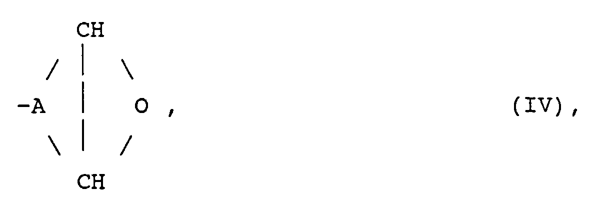 wobei **A** einen Alkyl-, Alkoxyalkyl-, Aryl- oder Alkarylrest bedeutet.

Beispiele für bevorzugte epoxyfunktionelle Reste **R'** sind

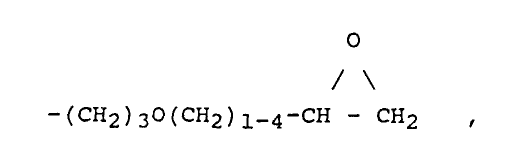

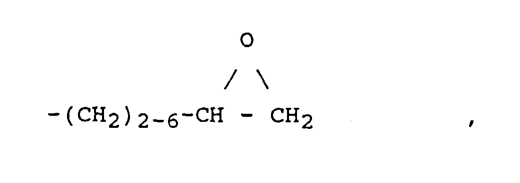

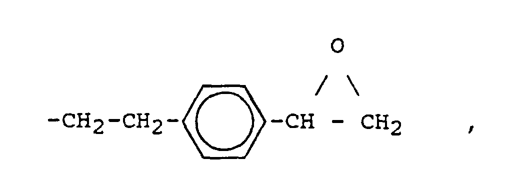

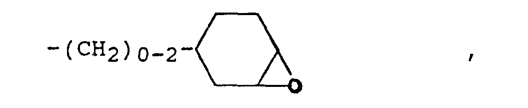

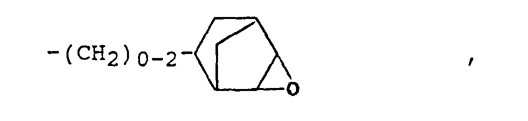

Besonders bevorzugte epoxyfunktionelle Reste **R'** sind

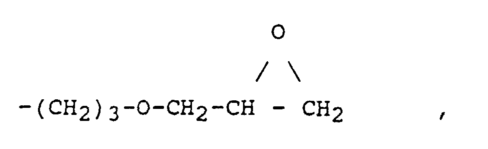

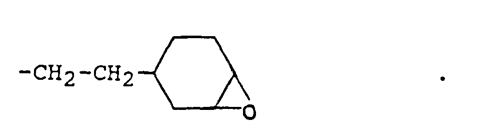

Die bevorzugten Epoxydzahlen der epoxyfunktionellen Organopolysiloxane (a) liegen bei 0,5 - 0,001 (equiv./100 g), insbesondere bei 0,2 - 0,01 (equiv./100 g). Die Epoxydzahl eines epoxyfunktionellen Organopolysiloxans gibt die Anzahl an Equivalenten an Epoxyd, nämlich die Molzahl an Epoxydgruppen an, die in 100 Gramm Organopolysiloxan (a) enthalten ist.

Beispiele für bevorzugte carboxyfunktionelle Reste **R'** sind Reste der allgemeinen Formel (V)

-X-(COOH)ₚ (V),

und deren durch Umsetzung mit Basen herstellbare Salze,
wobei
- **X**: einen linearen, verzweigten aliphatischen, aromatischen oder gemischt aliphatisch-aromatischen Kohlenwasserstoffrest bedeutet, dessen Kohlenstoffgerüst durch zweiwertige Schwefel-, Sauerstoff- oder Carbonsäureesterreste unterbrochen sein kann und
- **p**: den Wert 1 oder 2 hat.

Als carboxyfunktionelle Reste **R'** sind besonders bevorzugt die Reste

-(CH₂)₄₋₁₀-COOH ,

-CH₂CH(CH₃)-COOH ,

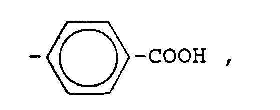

-CH₂CHR³-S-CH₂-COOH,

wobei R³ ein Wasserstoffatom, einen Methyl- oder Ethylrest bedeutet, 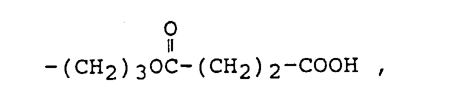 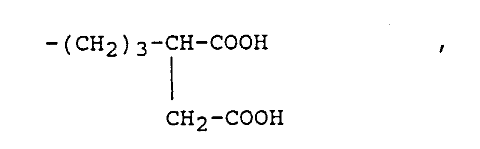 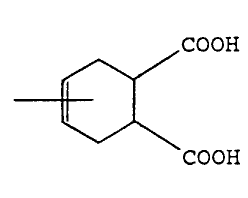 und

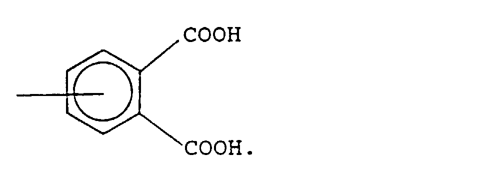

Insbesondere bevorzugt als carboxyfunktionelle Reste **R'** sind die Reste

-(CH₂)₁₀-COOH ,

-CH₂CH(CH₃)-COOH

und

-(CH₂)₂-S-CH₂-COOH .

Beispiele für Basen zur Umsetzung mit carboxyfunktionellen Resten **R'** aufweisenden Organopolysiloxanen (a) sind Ammoniak, Amine, Alkali- und Erdalkalihydroxide, wie LiOH, NaOH, KOH, RbOH, CsOH, Mg(OH)₂, Ca(OH)₂, Sr(OH)₂ und Ba(OH)₂.

Die bevorzugten Säurezahlen der carboxyfunktionellen Organopolysiloxane (a) liegen bei 1 - 100 (mg KOH/g), vorzugsweise bei 5 - 50 und insbesondere bei 10 - 30. Die Säurezahl eines carboxyfunktionellen Organopolysiloxans (a) gibt die Anzahl an Milligramm Kaliumhydroxid an, die notwendig ist, um die freien Säuren zu neutralisieren, die in einem Gramm des carboxyfunktionellen Organopolysiloxans (a) enthalten sind.

Vorzugsweise sind die Reste **R** Methyl-, Ethyl-, Phenyl-, Methoxy- und/oder Vinylreste. Wegen der leichteren Zugänglichkeit sind vorzugsweise 50 % der Reste **R**, insbesondere mindestens 80 % der Reste **R**, Methylreste.

Es kann ein Organopolysiloxan (a), vorzugsweise ein solches der Formel (I), eingesetzt werden; es können auch mehrere Organopolysiloxane eingesetzt werden.

Das in den erfindungsgemäßen Emulsionen eingesetzte Organopolysiloxan(gemisch) ist vorzugsweise flüssig. Insbesondere besitzen die im erfindungsgemäßen Verfahren eingesetzten Organopolysiloxane jeweils Viskositäten von 100 mPa*s bis 1.000.000 mPa*s, jeweils gemessen bei 25°C.

Wird ein aminofunktionelles Organopolysiloxan zur Herstellung des vorzugsweise in den erfindungsgemäßen Emulsionen eingesetzten ammoniumfunktionellen Organopolysiloxans (a) verwendet, so ist bevorzugt, daß es eine Aminzahl von 0,1 bis 3,0, insbesondere von 0,2 bis 0,9, besitzt. Die Aminzahl eines aminofunktionellen Stoffes wird bestimmt als Verbrauch in cm³ an 1n Salzsäure bei der Titration von 1 g des aminofunktionellen Stoffes.

Als Alkylpolyglykoside können beispielsweise die in der EP-A 418 479 beschriebenen Alkylpolyglykoside der allgemeinen Formel (VI)

R''-O-Zₒ , (VI)

eingesetzt werden,
wobei
- **R''**: einen linearen oder verzweigten, gesättigten oder ungesättigten Alkylrest mit im Mittel 8 bis 24 Kohlenstoffatomen, vorzugsweise 8 bis 16 Kohlenstoffatomen, und
- **Z**_{**o**}: einen Oligoglycosidrest mit im Mittel **o** = 1 bis 10, vorzugsweise 1 bis 5, Hexose- oder Pentoseeinheiten oder Gemischen davon bedeuten.

Besonders bevorzugt sind Alkylpolyglykoside mit gesättigtem Alkylrest mit im Mittel 8 bis 14 Kohlenstoffatomen und einem mittleren Glykosidierungsgrad n zwischen 1,1 und 3.

Die erfindungsgemäßen Emulsionen weisen vorzugsweise eine mittlere Teilchengröße von höchstens 1 µm, insbesondere von höchstens 300 nm auf. Die erfindungsgemäßen Mikroemulsionen weisen vorzugsweise eine mittlere Teilchengröße von höchstens 150 nm, insbesondere von höchstens 20 nm, auf. Der Ausdruck "Emulsionen" umfaßt im ganzen Text auch Mikroemulsionen. Der Ausdruck "Mikroemulsionen" bezieht sich nur auf Emulsionen mit einer mittleren Teilchengröße von höchstens 150 nm, die transparent bis optisch klar sind. Mikroemulsionen von Organopolysiloxanen mit Alkylpolyglykosiden als Emulgatoren sind nicht vorbeschrieben.

Beispielsweise zur Verkleinerung der Teilchengröße und zur Verringerung der benötigten Menge an Alkylpolyglycosiden (b) können die erfindungsgemäßen Emulsionen, insbesondere die Mikroemulsionen, auch Cotenside in Mengen von 0 bis 30 Gewichtsteilen, insbesondere höchstens 20 Gewichtsteilen, jeweils bezogen auf 100 Gewichtsteile der Organopolysiloxane (a), enthalten.

Unter Cotensiden versteht man polare Verbindungen mittlerer Molmasse, wie Alkohole der Molekülgröße C₄ bis C₈, geeignete Glykolether, Amine, Ester oder Ketone.

Beispiele für besonders geeignete Cotenside sind 1-Butanol, 2-Butanol, 2-Methyl-2-propanol, 1-Pentanol, 2-Pentanol, 3-Pentanol, 1-Hexanol, 2-Hexanol, 3-Hexanol, 1-Heptanol, 2-Heptanol, 3-Heptanol, 4-Heptanol, 1-Octanol, 2-Octanol, 3-Octanol und 4-Octanol; Diethylenglycolmonomethyl-, -ethyl-, und -butylether; Diethylenglycoldimethyl- und -ethylether; 1-Aminobutan, 2-Aminobutan, 2-Amino-2-methyl-propan, 1-Aminopentan, 2-Aminopentan, 1-Aminohexan, 1-Aminoheptan und 1-Aminooctan; Ethyl-, Propyl-, Isopropyl-, Butyl-, Isobutyl-, Pentyl-, Isopentyl- und Hexylacetat; Methyl-, Ethyl- und tert.-Butylpropionat; Methyl-, Ethyl-, Propyl- und Butylbutyrat; 2-Butanon, 2-Pentanon, 3-Pentanon, 4-Methyl-2-pentanon, 2-Hexanon, 3-Hexanon, 2-Heptanon, 3-Heptanon, 4-Heptanon, 5-Methyl-3-Heptanon, 2-Octanon und 3-Octanon.

Beispiele für bevorzugte Cotenside sind 1-Alkanole der vorstehend aufgeführten Beispiele mit C₅- bis C₈-Ketten, Diethylenglycolmonobutylether, Diethylenglycoldimethyl- und Diethylenglycoldiethylether, Propyl-, Butyl- und Pentylacetat sowie 2-Pentanon.

Als Cotenside besonders bevorzugt sind 1-Pentanol, 1-Hexanol und 1-Octanol, Diethylenglycolmonobutylether, Diethylenglycoldimethylether und Butylacetat.

Außer Organopolysiloxan (a), Alkylpolyglykosiden (b), Wasser und gegebenenfalls Cotensid können die erfindungsgemäßen Emulsionen noch Zusatzstoffe enthalten. Die sind insbesondere Bactericide, Fungicide, Algicide, Mikrobicide, Duftstoffe, Korrosionsinhibitoren, Farbstoffe, Pigmente, Verdickungsmittel und Füllstoffe. Die erfindungsgemäßen Emulsionen enthalten Zusatzstoffe vorzugsweise in Mengen von 0 bis 1 Gewichtsprozent, insbesondere von 0 bis 0,2 Gewichtsprozent, jeweils bezogen auf das Gesamtgewicht der fertigen Emulsion.

Das Vermischen aller Komponenten der erfindungsgemäßen Emulsion kann in beliebiger Reihenfolge unter Verwendung von Emulgiergeräten oder durch Zusammenrühren ohne Aufbietung hoher Scherkräfte erfolgen. Es ist jedoch bevorzugt, daß zuerst eine homogene Mischung von Organopolysiloxan (a), Alkylpolyglykosiden (b) und Wasser hergestellt und in diese Mischung die Cotenside und Zusatzstoffe, sofern eingesetzt, ohne Aufbietung hoher Scherkräfte eingerührt werden.

Der auf die jeweiligen Komponenten bzw. Mischungen ausgeübte Druck ist vorzugsweise der gegebenenfalls durch Einwirkung der Mischorgane erhöhte (Atmosphären-) Druck; die entsprechend herrschende Temperatur ist vorzugsweise die gegebenenfalls durch Einwirkung der Mischorgane erhöhte (Raum-)Temperatur.

Das vorzugsweise in den erfindungsgemäßen Emulsionen enthaltene eingesetzte ammoniumfunktionelle Organopolysiloxan (a) kann hergestellt werden durch Zugabe von Mineralsäuren oder Carbonsäuren zu entsprechenden aminofunktionellen Organopolysiloxanen. Diese Zugabe von Säure zum Organopolysiloxan (a) kann erfolgen, bevor das Organopolysiloxan (a) eingesetzt wird.

In einer besonders bevorzugten Ausführungsform zur Herstellung der erfindungsgemäßen Emulsionen unter Einsatz besonders bevorzugter amino- und/oder ammoniumfunktioneller Organopolysiloxane (a) werden die ammoniumfunktionellen Reste jedoch in situ beim Mischen von Organopolysiloxan (a), Alkylpolyglykosiden (b) und Wasser durch Zugabe der vorstehend beschriebenen Mineral- und/oder Carbonsäuren, insbesondere Essigsäure, erzeugt.

Die erfindungsgemäßen Emulsionen können grundsätzlich in jedem turbulenten Mischer hergestellt werden, der auch bisher zur Herstellung von Emulsionen verwendet wurde. Beispiele für vervendbare Mischer sind Rührer, wie Blatt-, Balken-, Anker-, Gitter-, Schnecken-, Propeller-, Scheiben-, Impeller-, Turbinen-, Planetenrührer, Ein- und Doppelschneckenmischer, Mischturbinen, Kolloidmühlen, Ultraschallmischer, In-line-Mischer, Pumpen, Homogenisatoren, wie Hochdruck-, Turbinen- und Umlaufhomogenisatoren.

Die erfindungsgemäßen Emulsionen können überall eingesetzt werden wo auch bisher Siliconemulsionen eingesetzt worden sind. Besonders sind sie geeignet als Mittel oder als Bestandteil eines Mittels zur Imprägnierung von Fasern und Geweben, in Kosmetika, Reinigungs- und Poliermitteln, in Anstrichen oder Imprägniermitteln für Baustoffe und deren Vorprodukte, in Antischaummitteln und für klebrige Stoffe abweisende Beschichtungen. So können sie verwendet werden zum Schlichten von Glas-, Keramik- und Kohlefasern, zum Imprägnieren und Beschichten von Textilfasern - beispielsweise als Fadengleitmittel - und Textilgeweben, in Kosmetika, wie Handcremes, Body-Lotions, Shampoos, Haarspülungen, Haarfestigern, Rasiercremes und -lotionen, in Polituren, wie Möbel-, Fußboden- und Autopolituren, in Wachsen, wie Fußbodenwachsen und in Desinfektionsmitteln, zur Hydrophobierung von Gips vor oder nach dessen Formgebung zu Bauteilen, zum Imprägnieren von Natur- oder Kunststein, Beton, Zement, Mauerwerk, zum Hydrophobieren von Gasbeton vor oder nach dessen Aufschäumen, in Anstrichen für Bauwerke und deren Teile, wie Dispersionsfarben, insbesondere in Siliconfarben, in oder als Papierbeschichtungen für Träger von selbstklebenden Etiketten und als Formtrennmittel für Polymere.

Besonders bevorzugt ist die Verwendung der erfindungsgemäßen Emulsionen, insbesondere der Emulsionen aminofunktioneller und ammoniumfunktioneller Organopolysiloxane (a), als Mittel oder in Mitteln zur Imprägnierung und Beschichtung von Textilfasern und -geweben. So verleihen die erfindungsgemäßen Emulsionen den damit behandelten textilen Fasern und Flächengebilden beispielsweise einen sehr angenehmen, weichen Griff.

### Beispiele

In den nachfolgenden Beispielen sind, soweit nichts anderes angegeben ist, alle Mengen- und Prozentangaben auf das Gewicht bezogen. Falls jeweils nicht anders angegeben, werden die nachfolgenden Beispiele bei Atmosphärendruck (etwa 0,1 MPa (abs.)) und Raumtemperatur (etwa 20°C) bzw. bei Temperaturen und Drücken, die sich beim Zusammengeben der Reaktanden bei Raumtemperatur ohne zusätzliche Heizung oder Kühlung oder durch die Einwirkung von Mischorganen auf die Komponenten bzw. Mischungen einstellen, durchgeführt.

### Aminofunktionelle Organopolysiloxane (a):

### Polysiloxan A1:

Organopolysiloxan bestehend aus Dimethylsiloxy-Einheiten, Methyl(N-[2-Aminoethyl]-3-aminopropyl)siloxy-Einheiten und endständigen Methoxydimethylsilylgruppen; Viskosität: 1000 mPa.s bei 25°C; Aminzahl: 0.3.

### Polysiloxan A2:

Organopolysiloxan bestehend aus Dimethylsiloxy-Einheiten, Methyl(N-[2-Aminoethyl]-3-aminopropyl)siloxy-Einheiten und endständigen Methoxydimethylsilylgruppen; Viskosität: 6500 mPa.s bei 25°C; Aminzahl: 0.13.

### Polysiloxan A3:

Organopolysiloxan bestehend aus Dimethylsiloxy-Einheiten, Methyl(N-[2-Aminoethyl]-3-aminopropyl)siloxy-Einheiten und endständigen Methoxydimethylsilylgruppen; Viskosität: 1200 mPa.s bei 25°C; Aminzahl: 0.6.

### Carboxyfunktionelles Organopolysiloxan A4:

Organopolysiloxan bestehend aus Dimethylsiloxy-Einheiten, Methyl(2-Carboxypropyl)siloxy-Einheiten und endständigen Trimethylsilylgruppen; Viskosität: 2000 mPa.s bei 25°C; Säurezahl: 17.

### Epoxyfunktionelles Organopolysiloxan A5:

Organopolysiloxan bestehend aus Dimethylsiloxy-Einheiten, Methyl(3-Glycidyloxypropyl)siloxy-Einheiten und endständigen Trimethylsilylgruppen; Viskosität: 8000 mPa.s bei 25°C; Epoxidzahl: 0.026.

### Emulgator B:

50 %ige Lösung in Wasser eines C₈/C₁₁-Alkylpolyglykosids mit einem Glycosidierungsgrad von 1.35, käuflich unter dem Warenzeichen Atlas G-2541 bei ICI Specialty Chemicals.

### Beispiel 1

30 g Emulgator B, 0,45 g Eisessig und 42,5 g Polysiloxan A1 werden mit einem Ultra-Turrax^{R} T 45 der Fa. Janke und Kunkel homogenisiert. In die resultierende homogene Paste werden anschließend 192 g vollentsalztes Wasser (VE-Wasser) mit dem Ultra-Turrax^{R} eingearbeitet. Es wird eine niederviskose, feinteilige (mittlerer Teilchendurchmesser 200 nm) Emulsion mit einem Silicongehalt von 16 % erhalten, die nach einer Standzeit von mehr als 6 Monaten keinerlei Anzeichen von Instabilität zeigt und eine hervorragende Stabilität gegen Fremdelektrolytzusatz aufweist.

### Beispiel 2

265 g Emulsion aus Beispiel 1 werden unter einfachem Rühren ohne Aufbietung hoher Scherkräfte mit 4 g 1-Pentanol versetzt. Es resultiert eine niederviskose, klare Mikroemulsion (mittlerer Teilchendurchmesser 7 nm) mit einem Silicongehalt von 15,8 %, die nach einer Standzeit von mehr als 6 Monaten keinerlei Anzeichen von Instabilität zeigt.

Aufzeigen der hervorragenden Stabilität der Mikroemulsion aus Beispiel 2 gegen Fremdelektrolytzusatz:
a) 20 g Mikroemulsion aus Beispiel 2 werden im zeitlichen Abstand von 5 Minuten portionsweise unter Rühren bzw. Schütteln mit jeweils 200 mg festem Natriumchlorid versetzt. Erst nach einer Gesamtzugabe von 3,6 g Natriumchlorid innerhalb von 90 Minuten beginnt die bis dahin vollkommen klare Mikroemulsion trübe zu werden.
b) 2 g Mikroemulsion aus Beispiel 2 werden mit 18 g VE-Wasser verdünnt und die resultierende klare Mikroemulsion wie in a) portionsweise mit festem Natriumchlorid versetzt. Nach einer Gesamtzugabe von 7,0 g Natriumchlorid innerhalb von 175 Minuten wird die Löslichkeitsgrenze für Natriumchlorid überschritten; die Mikroemulsion ist weiterhin klar.

### Vergleichsbeispiel 1:

Es wird in Analogie zu Beispiel 1 verfahren mit der Abänderung, daß anstelle von 30 g Emulgator B 15 g eines Isotridecylalkoholpolyglykolethers mit 6 Ethylenoxyeinheiten (100 %ig), käuflich als Genapol^{R} X060 bei der Hoechst AG, verwendet werden. 265 g der resultierenden Emulsion werden wie in Beispiel 2 unter Rühren mit 4 g 1-Pentanol versetzt und die Stabilität der resultierenden klaren Mikroemulsion gegen Fremdelektrolytzusatz wie in Beispiel 2 untersucht.
a) 20 g Mikroemulsion aus Vergleichsbeispiel 1 werden im zeitlichen Abstand von 5 Minuten portionsweise unter Rühren bzw. Schütteln mit jeweils 200 mg festem Natriumchlorid versetzt. Bereits nach einer Gesamtzugabe von 0,4 g Natriumchlorid innerhalb von 10 Minuten zeigt die ursprünglich klare Mikroemulsion intensive Weißfärbung und trennt sich im Verlauf von 16 Stunden in zwei Phasen.
b) 2 g Mikroemulsion aus Vergleichsbeispiel 1 werden mit 18 g VE-Wasser verdünnt und die resultierende klare Mikroemulsion wie in a) mit festem Natriumchlorid versetzt. Bereits nach einer Gesamtzugabe von 0,8 g Natriumchlorid innerhalb von 20 Minuten erfolgt Trübung.

### Beispiel 3

30 g Emulgator B, 0,38 g Eisessig und 82,5 g Polysiloxan A2 werden mit einem Ultra-Turrax^{R} T 45 der Fa. Janke und Kunkel homogenisiert. In die resultierende homogene Paste werden anschließend 139,5 g VE-Wasser mit dem Ultra-Turrax^{R} eingearbeitet. Es wird eine niederviskose, feinteilige (mittlerer Teilchendurchmesser 250 nm) Emulsion mit einem Silicongehalt von 32,7 % erhalten, die nach einer Standzeit von mehr als 6 Monaten keinerlei Anzeichen von Instabilität zeigt und eine gute Stabilität gegen Fremdelektrolytzusatz aufweist.

### Beispiel 4

30 g Emulgator B, 0,98 g Eisessig und 42,5 g Polysiloxan A3 werden mit einem Ultra-Turrax^{R} T 45 der Fa. Janke und Kunkel homogenisiert. In die resultierende homogene Paste werden anschließend 191,5 g VE-Wasser mit dem Ultra-Turrax^{R} eingearbeitet. Es wird eine niederviskose, transparente Mikroemulsion (mittlerer Teilchendurchmesser 51 nm) mit einem Silicongehalt von 16 % erhalten, die nach einer Standzeit von mehr als 6 Monaten keinerlei Anzeichen von Instabilität zeigt und eine gute Stabilität gegen Fremdelektrolytzusatz aufweist.

### Beispiel 5

100 g Emulsion aus Beispiel 4 werden unter einfachem Rühren ohne Aufbietung hoher Scherkräfte mit 2,3 g Diethylenglycolmonobutylether versetzt. Es resultiert eine niederviskose, klare Mikroemulsion (mittlerer Teilchendurchmesser 15 nm) mit einem Silicongehalt von 15,6 %, die nach einer Standzeit von mehr als 6 Monaten keinerlei Anzeichen von Instabilität zeigt und eine sehr gute Stabilität gegen Fremdelektrolytzusatz aufweist.

### Beispiel 6:

30 g Emulgator B, 192 g VE-Wasser, 42,5 g Polysiloxan A1 und 0,45 g Eisessig werden 1 Stunde lang ohne Aufbietung hoher Scherkräfte am Drehkreuz vermischt und anschließend unter Rühren ohne Aufbietung hoher Scherkräfte mit 8 g 1-Pentanol versetzt. Es resultiert eine niederviskose, klare Mikroemulsion (mittlerer Teilchendurchmesser 15 nm) mit einem Silicongehalt von 15,5 %, die nach einer Standzeit von mehr als 6 Monaten keinerlei Anzeichen von Instabilität zeigt und eine gute Stabilität gegen Fremdelektrolytzusatz aufweist.

### Beispiel 7

30 g Emulgator B, 42,5 g Polysiloxan A4 und 15,7 g wäßrige Kaliumhydroxidlösung (0,7 molar) werden mit einem Ultra-Turrax^{R} T 50 der Fa. Janke und Kunkel homogenisiert. In die resultierende homogene Paste werden anschließend 160 g VE-Wasser mit dem Ultra-Turrax^{R} eingearbeitet. Es wird eine niederviskose, stabile, transparente Mikroemulsion (mittlerer Teilchendurchmesser 28 nm) mit einem Silicongehalt von 17,1 % erhalten.

Aufzeigen der sehr guten Stabilität der Mikroemulsion aus Beispiel 7 gegen Fremdelektrolytzusatz:

2 g Mikroemulsion aus Beispiel 7 werden mit 18 g VE-Wasser verdünnt und die resultierende fast klare Mikroemulsion unter Rühren bzw. Schütteln mit insgesamt 8 g festem Natriumchlorid versetzt, wobei die Löslichkeitsgrenze für Natriumchlorid überschritten wird; die Mikroemulsion ist auch nach 24 Stunden unverändert fast klar.

### Vergleichsbeispiel 2:

Es wird in Analogie zu Beispiel 7 verfahren mit der Abänderung, daß anstelle von 30 g Emulgator B 19 g eines Isotridecylalkoholpolyglykolethers mit 10 Ethylenoxyeinheiten (80 %ig in Wasser), käuflich als Arlypon^{R} IT 10/80 bei der Chemischen Fabrik Grünau GmbH, im Gemisch mit 11 g VE-Wasser eingesetzt werden. Es wird eine niederviskose, stabile, leicht trübe Mikroemulsion (mittlerer Teilchendurchmesser 68 nm) mit einem Silicongehalt von 17,1 % erhalten und ihre Stabilität gegen Fremdelektrolytzusatz wie in Beispiel 7 untersucht.

2 g der Mikroemulsion aus Vergleichsbeispiel 2 werden mit 18 g VE-Wasser verdünnt und die resultierende leicht trübe Mikroemulsion wird wie in Beispiel 7 unter Rühren bzw. Schütteln mit insgesamt 8 g festem Natriumchlorid versetzt, wobei die Löslichkeitsgrenze für Natriumchlorid überschritten wird; eine spontane Zunahme der Trübung ist nicht festzustellen. Nach 24 Stunden jedoch beobachtet man Ölabscheidung und eine deutliche Zunahme der Trübung der wäßrigen Phase.

### Beispiel 8

30 g Emulgator B und 42,5 g Polysiloxan A5 werden mit einem U1tra-Turrax^{R} T 50 der Fa. Janke und Kunkel homogenisiert. In die resultierende homogene Paste werden anschließend 175 g VE-Wasser mit dem Ultra-Turrax^{R} eingearbeitet. Es wird eine niederviskose, stabile Emulsion mit einem Silicongehalt von 17,2 % und einem mittleren Teilchendurchmesser von 370 nm erhalten.

### Vergleichsbeispiel 3

In Vergleichsbeispiel 3 wird eine nicht erfindungsgemäße Emulsion eines Polydimethylsiloxanöles mit einem Alkylpolyglykosid (3a) einer Emulsion des gleichen Öles unter Verwendung eines Alkoholethoxylats (3b) gegenübergestellt und die Stabilitäten der Emulsionen aus 3a) und 3b) gegen Fremdelektrolytzusatz verglichen. Vergleichsbeispiel 3a) orientiert sich an Beispiel 1 aus EP-A 418 479.
a) 23 g Emulgator B und 50 g eines Polydimethylsiloxanöles mit endständigen Trimethylsilylgruppen und einer Viskosität von 500 mPa.s werden mit einem Ultra-Turrax^{R} T 50 der Fa. Janke und Kunkel homogenisiert; in die resultierende homogene Paste werden anschließend 427 g VE-Wasser mit dem Ultra-Turrax^{R} eingearbeitet. Es wird eine niederviskose, feinteilige (mittlerer Teilchendurchmesser 280 nm), stabile Emulsion mit einem Silicongehalt von 10,0 % erhalten.
b) Es wird in Analogie zu Vergleichsbeispiel 3a) verfahren mit der Abänderung, daß anstelle von 23 g Emulgator B 14,5 g einer 80 %igen Lösung in Wasser eines Isotridecylalkoholpolyglykolethers mit 10 Ethylenoxyeinheiten, käuflich als Arlypon^{R} IT 10/80 bei der Chemischen Fabrik Grünau GmbH, im Gemisch mit 8,5 g VE-Wasser eingesetzt werden. Es wird eine niederviskose, feinteilige (mittlerer Teilchendurchmeseer 240 nm), stabile Emulsion mit einem Silicongehalt von 10,0 % erhalten.

Untersuchung der Stabilität der Emulsionen aus Vergleichsbeispielen 3a) und 3b) gegen Fremdelektrolytzusatz: Je 20 g Emulsion aus Vergleichsbeispielen 3a) und 3b) sowie je 20 g einer 1:10-Verdünnung dieser Emulsionen werden portionsweise unter Schütteln mit jeweils insgesamt 7 g Natriumchlorid bis zum Erreichen der Löslichkeitsgrenze von Natriumchlorid versetzt. Eine spontane Veränderung der bereits vor Beginn der Elektrolytzugabe weißen Emulsionen während der Natriumchloridzugabe ist in keinem Fall festzustellen. 5 Stunden nach Beendigung der Salzzugabe beobachtet man in allen Fällen Ölabscheidungen.

Die Emulsionen aus Vergleichsbeispiel 3a) und 3b) weisen keine Unterschiede hinsichtlich ihrer Stabilität gegen Elektrolytzusatz auf.

## Patentansprüche (Patentansprüche für folgende(n) Vertragsstaat(en): AT, BE, CH, FR, GB, IT, NL)

1. Wäßrige Emulsionen auf der Grundlage von
a) 100 Gewichtsteilen an Organopolysiloxanen, welche polare Gruppen an Si-C gebundenen Kohlenwasserstoffresten aufweisen, mit der Maßgabe, daß Methyl- oder Phenylgruppen nicht unter die polare Gruppen aufweisenden Si-C-gebundenen Kohlenwasserstoffreste zu rechnen sind, und
b) höchstens 50 Gewichtsteilen an Alkylpolyglykosiden.

2. Emulsionen nach Anspruch 1, wobei die Organopolysiloxane (a) die allgemeine Formel (I)
RₙR'ₘSiO_{(4-n-m)/2} (I)
aufweisen, worin
**R** gleiche oder verschiedene, gegebenenfalls substituierte Kohlenwasserstoffreste oder Kohlenwasserstoffoxyreste mit jeweils 1 bis 18 Kohlenstoffatomen, Wasserstoffatome oder Hydroxyreste,
**R'** gleiche oder verschiedene, Si-C-gebundene, polare Gruppen, mit der Maßgabe, daß die polaren Gruppen keine Methyl- oder Phenylgruppen sind, enthaltende substituierte Kohlenwasserstoffreste,
**n** eine ganze Zahl im Wert von 0, 1, 2 oder 3,
**m** eine ganze Zahl im Wert von 0, 1, 2 oder 3
bedeuten, und die Summe n+m einen durchschnittlichen Wert von 1,8 bis 2,2 besitzt und m so gewählt ist, daß das Polyorganosiloxan mindestens einen Rest R' aufweist.

3. Emulsionen nach Anspruch 1 oder 2, wobei die polaren an Si-C-gebundenen Kohlenwasserstoffresten gebundenen Gruppen Amino-, Ammonium-, Epoxy-, Hydroxy-, Amido-, Mercapto-, Carboxy- und/oder Sulfonsäuregruppen, deren Ester oder Salze sind.

4. Emulsionen nach Anspruch 1, 2, oder 3, welche zusätzlich Cotenside enthalten.

## Patentansprüche (Patentansprüche für folgende(n) Vertragsstaat(en): DE)

1. Wäßrige Emulsionen auf der Grundlage von
a) 100 Gewichtsteilen an Organopolysiloxanen, welche polare Gruppen an Si-C-gebundenen Kohlenwasserstoffresten aufweisen, mit der Maßgabe, daß die polaren Gruppen keine Amino- oder Amidogruppe sind und daß Methyl- oder Phenylgruppen nicht unter die polare Gruppen aufweisenden Si-C-gebundenen Kohlenwasserstoffreste zu rechnen sind, und
b) höchstens 50 Gewichtsteilen an Alkylpolyglykosiden.

2. Emulsionen nach Anspruch 1, wobei die Organopolysiloxane (a) die allgemeine Formel (I)
RₙR'ₘSiO_{(4-n-m)/2} (I)
aufweisen, worin
**R** gleiche oder verschiedene, gegebenenfalls substituierte Kohlenwasserstoffreste oder Kohlenwasserstoffoxyreste mit jeweils 1 bis 18 Kohlenstoffatomen, Wasserstoffatome oder Hydroxyreste,
**R'** gleiche oder verschiedene, Si-C-gebundene, polare Gruppen, mit der Maßgabe, daß die polaren Gruppen keine Methyl- oder Phenylgruppen sind, enthaltende substituierte Kohlenwasserstoffreste,
**n** eine ganze Zahl im Wert von 0, 1, 2 oder 3,
**m** eine ganze Zahl im Wert von 0, 1, 2 oder 3
bedeuten, und die Summe n+m einen durchschnittlichen Wert von 1,8 bis 2,2 besitzt und m so gewählt ist, daß das Polyorganosiloxan mindestens einen Rest **R'** aufweist.

3. Emulsionen nach Anspruch 1 oder 2, wobei die polaren an Si-C-gebundene Kohlenwasserstoffreste gebundenen Gruppen Ammonium-, Epoxy-, Hydroxy-, Mercapto-, Carboxy- und/oder Sulfonsäuregruppen, deren Ester oder Salze sind.

4. Emulsionen nach Anspruch 1, 2 oder 3, welche zusätzlich Cotenside enthalten.

## Claims (Claims for the following Contracting State(s): AT, BE, CH, FR, GB, IT, LI, NL)

1. Aqueous mulsions based on
a) 100 parts by weight of organopolysiloxanes which contain polar groups on Si-C-bonded hydrocarbon radicals, with the proviso that methyl or phenyl groups are not to be included with the SI-C-bonded hydrocarbon radicals which contain polar groups, and
b) not more than 50 parts by weight of alkyl polyglycosides.

2. Emulsions according to Claim 1, in which the organopolysiloxanes (a) have the general formula (I)
RₙR'ₘSiO_{(4-n-m)/2} (I)
in which
R denotes identical or different, optionally substituted hydrocarbon radicals or hydrocarbon-oxy radicals having in each case 1 to 18 carbon atoms, hydrogen atoms or hydroxyl radicals,
R' denotes identical or different Si-C-bonded substituted hydrocarbon radicals containing polar groups, with the proviso that the polar groups are not methyl or phenyl groups,
n denotes an integer having the value 0, 1, 2 or 3,
m denotes an integer having the value 0, 1, 2 or 3
and the sum n+m has an average value of 1.8 to 2.2, and m is chosen such that the polyorganosiloxane contains at least one radical R'.

3. Emulsions according to Claim 1 or 2, in which the polar groups bonded to Si-C-bonded hydrocarbon radicals are amino, ammonium, epoxide, hydroxyl, amido, mercapto, carboxyl and/or sulphonic acid groups and esters or salts thereof.

4. Emulsions according to Claim 1, 2, or 3, which additionally comprise cosurfactants.

## Claims (Claims for the following Contracting State(s): DE)

1. Aqueous emulsions based on
a) 100 parts by weight of organopolysiloxanes which contain polar groups on Si-C-bonded hydrocarbon radicals, with the proviso that the polar groups are not an amino or amido group, and that methyl or phenyl groups are not to be included with the Si-C-bonded hydrocarbon radicals which contain polar groups, and
b) not more than 50 parts by weight of alkyl polyglycosides.

2. Emulsions according to Claim 1, in which the organopolysiloxanes (a) have the general formula (I)
RₙR'ₘSiO_{(4-n-m)/2} (I)
in which
R denotes identical or different, optionally substituted hydrocarbon radicals or hydrocarbon-oxy radicals having in each case 1 to 18 carbon atoms, hydrogen atoms or hydroxyl radicals,
R' denotes identical or different Si-C-bonded substituted hydrocarbon radicals containing polar groups, with the proviso that the polar groups are not methyl or phenyl groups,
n denotes an integer having the value 0, 1, 2 or 3,
m denotes an integer having the value 0, 1, 2 or 3
and the sum n+m has an average value of 1.8 to 2.2, and m is chosen such that the polyorganosiloxane contains at least one radical R'.

3. Emulsions according to Claim 1 or 2, in which the polar groups bonded to Si-C-bonded hydrocarbon radicals are ammonium, epoxide, hydroxyl, mercapto, carboxyl and/or sulphonic acid groups and esters or salts thereof.

4. Emulsions according to Claim 1, 2, or 3, which additionally comprise cosurfactants.

## Revendications (Revendications pour l'(les) Etat(s) contractant(s) suivant(s): AT, BE, CH, FR, GB, IT, LI, NL)

1. Emulsion aqueuse à base de
a) 100 parties en poids d'un organopolysiloxane, qui présente des radicaux hydrocarbure à liaison Si-C, liés à des groupes polaires, avec la condition que les groupes méthyle ou phényle ne sont pas admis comme radicaux hydrocarbure à liaison Si-C présentant des groupes polaires, et
b) au plus 50 parties en poids d'un alcoylpolyglycoside.

2. Emulsion suivant la revendication 1, où l'organopolysiloxane
a) présente la formule générale (I)
RₙR'ₘSiO_{(4-n-m)/2} (I)
où
R représente des radicaux hydrocarbure ou des radicaux oxyhydrocarbure identiques ou différents, facultativement substitués, avec chacun 1 à 18 atomes de carbone, un atome d'hydrogène ou un radical hydroxyle,
R' représente des radicaux hydrocarbure substitués contenant des groupes polaires, identiques ou différents, à liaison Si-C, avec la condition que les groupes polaires ne soient pas des groupes méthyle ou phényle,
n représente un nombre entier de valeur 0, 1, 2 ou 3,
m représente un nombre entier de valeur 0, 1, 2 ou 3,
et la somme n+m possède une valeur moyenne de 1,8 à 2,2 et m est choisi de sorte que le polyorganosiloxane présente au moins un radical R'.

3. Emulsion suivant la revendication 1 ou 2, où les groupes polaires, liés aux radicaux hydrocarbure à liaison Si-C sont des groupes amino, ammonium, époxy, hydroxyle, amido, mercapto, carboxy et/ou acide sulfonique, leurs esters ou sels.

4. Emulsion suivant la revendication 1, 2 ou 3, qui contient en outre, un tensioactif.

## Revendications (Revendications pour l'(les) Etat(s) contractant(s) suivant(s): DE)

1. Emulsion aqueuse à base de
a) 100 parties en poids d'un organopolysiloxane, qui présente des radicaux hydrocarbure à liaison Si-C, liés à des groupes polaires, avec la condition que les groupes polaires ne sont pas des groupes amino ou amido et avec la condition que les groupes méthyle ou phényle ne sont pas admis comme radicaux hydrocarbure à liaison Si-C présentant des groupes polaires, et
b) au plus 50 parties en poids d'un alcoylpolyglycoside.

2. Emulsion suivant la revendication 1, où l'organopolysiloxane
a) présente la formule générale (I)
RₙR'ₘSiO_{(4-n-m)/2} (I)
où
R représente des radicaux hydrocarbure ou des radicaux oxyhydrocarbure identiques ou différents, facultativement substitués, avec chacun 1 à 18 atomes de carbone, un atome d'hydrogène ou un radical hydroxyle,
R' représente des radicaux hydrocarbure susbtitués contenant des groupes polaires, identiques ou différents, à liaison Si-C, avec la condition que les groupes polaires ne soient pas des groupes méthyle ou phényle,
n représente un nombre entier de valeur 0, 1, 2 ou 3,
m représente un nombre entier de valeur 0, 1, 2 ou 3,
et la somme n+m possède une valeur moyenne de 1,8 à 2,2 et m est choisi de sorte que le polyorganosiloxane présente au moins un radical R'.

3. Emulsion suivant la revendication 1 ou 2, où les groupes polaires, liés aux radicaux hydrocarbure à liaison Si-C sont des groupes ammonium, époxy, hydroxyle, mercapto, carboxy et/ou acide sulfonique, leurs esters ou sels.

4. Emulsion suivant la revendication 1, 2 ou 3, qui contient en outre, un tensioactif.
